# EUROPEAN PATENT APPLICATION

(11) **EP 4 675 637 A1**
(43) Date of publication of application: **07.01.2026**
(21) Application number: 25185508.6
(22) Date of filing: 26.06.2025
(51) Int. Cl.: G16H 40/63, G16H 40/67

(54) **ANNOTATION COLLECTING SYSTEM, AND ANNOTATION COLLECTING METHOD**

(30) Priority: 03.07.2024 JP 2024107269
(71) Applicant: Nihon Kohden Corporation, Shinjuku-ku Tokyo (JP); Tokyo Metropolitan Hospital Organization, Shinjuku-ku Tokyo (JP)
(72) Inventor: Terai, Kumi, Tokorozawa-shi, Saitama (JP); Matsuzawa, Wataru, Tokorozawa-shi, Saitama (JP); Hayashi, Satoshi, Tokorozawa-shi, Saitama (JP); Sano, Hiroto, Tokorozawa-shi, Saitama (JP); Akahoshi, Shogo, Shinjuku-ku, Tokyo (JP); Morikawa, Yoshihiko, Shinjuku-ku, Tokyo (JP); Honda, Masataka, Shinjuku-ku, Tokyo (JP)
(74) Representative: Grünecker Patent- und Rechtsanwälte PartG mbB

(57) **Abstract**

A medical device is configured to issue an alert. An annotation assigning device is configured to provide an input user interface arranged to input managing information indicating whether the alert is an actionable alarm or a non-actionable alarm. The annotation assigning device is configured to assign an annotation corresponding to the managing information to the alert.

## Description

### BACKGROUND

The invention relates to an annotation collecting system and an annotation collecting method that are for inputting information indicative how to manage an alert issued from a medical device.

Japanese Patent Publication No. 2021-070257A discloses a monitor device configured to collectively display physiological information of a subject such as a patient, as an example of the medical device described above. The monitor device is configured to issue an alert when an abnormality occurs in a measured value or the like of the physiological information.

### SUMMARY

It is required to reduce the burden on medical workers managing the alerts.

An illustrative aspect of the present disclosure may provide an annotation collecting system, comprising:
a medical device configured to issue an alert; and
an annotation assigning device configured to provide an input user interface arranged to input managing information indicating whether the alert is an actionable alarm or a non-actionable alarm, and to assign an annotation corresponding to the managing information to the alert.

An illustrative aspect of the present disclosure may provide an annotation collecting method, comprising:
providing an input user interface arranged to input managing information indicating whether an alert issued from a medical device is an actionable alarm or a non-actionable alarm; and
assigning an annotation corresponding to the managing information to the alert.

For example, even in a situation where an alarm indicating tachycardia is issued from the medical device with respect to a certain subject, there would be a case where tachycardia that requires treatment actually occurs (actionable) and a case where no treatment is required because a temporary pulse increase is caused by crying or erroneous detection of the sensor (non-actionable). As long as the possibility of an actionable alarm is undeniable, medical workers are required to check how the alert is triggered. However, the frequency of the non-actionable alarm is higher than the frequency of the actionable alarm. As the number of sensors and medical devices for monitoring a subject increases, the number of managements to non-actionable alarms with a relatively low urgency tends to increase as well. Accordingly, the burden on medical workers called "alarm fatigue" tends to increase.

Thus, it is required for the medical device to have a capability of appropriately discriminating the actionable alarm from the non-actionable alarm, and suppressing the output of the latter. It is conceivable that the medical device is provided with an inference engine having acquired a discrimination capability through machine learning. However, in order to prepare an inference engine having a highly accurate discrimination capability, an enormous amount of training data is required. The training data is, for example, a combination of physiological information that triggers an alert to be issued, and an annotation indicating whether the alert is an actionable alarm or a non-actionable alarm.

According to the configuration of each of the above illustrative aspects, since the medical worker himself/herself who has managed the alert can assign an annotation to the alert through the input user interface, it is possible to prompt efficient collection of annotations having strongly reflected the monitoring environment of the physiological information in a workplace of the medical worker. Especially, since it is prompted to accumulate cases related to the non-actionable alarms issued frequently, it is possible to contribute to preparation of the training data with high quality. As a result, it is possible to reduce the burden imposed on the medical workers managing the alerts.

### DESCRIPTION OF THE DRAWINGS

FIG. 1 illustrates an entire configuration of an annotation collecting system according to an exemplary embodiment.
FIG. 2 illustrates a functional configuration of the annotation collecting system of FIG. 1.
FIG. 3 illustrates one exemplary appearance of an input user interface of FIG. 2.
FIG. 4 illustrates another exemplary appearance of the input user interface of FIG. 2.
FIG. 5 illustrates a processing flow performed by an annotation assigning device of FIG. 2.
FIG. 6 illustrates an appearance of an auxiliary input user interface.

### DESCRIPTION OF EMBODIMENTS

Exemplary embodiments will be described in detail with reference to the accompanying drawings.

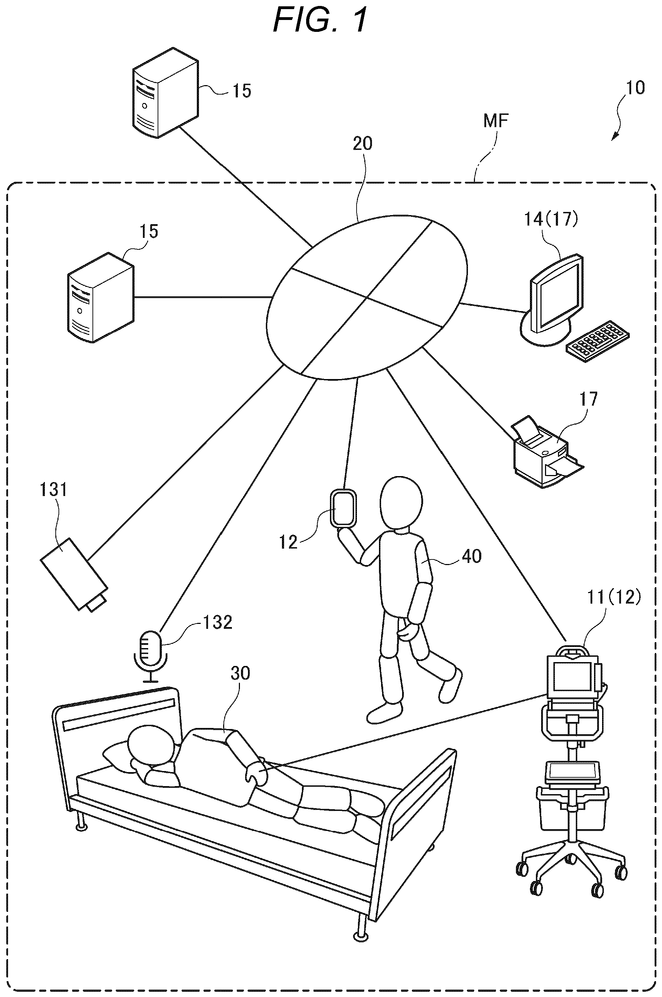

FIG. 1 illustrates a configuration of an annotation collecting system 10 according to an exemplary embodiment. The collecting system 10 includes a monitor device 11 and an annotation assigning device 12 (hereinafter, abbreviated as "an assigning device 12"). The monitor device 11 and the assigning device 12 are communicably connected via a communication network 20.

The monitor device 11 is a device that acquires physiological information of a subject 30 by way of a sensor (not illustrated). The sensor may be configured to be attached to the subject 30 to acquire the physiological information, or may be configured to acquire the physiological information without contact. Examples of the physiological information include an electrocardiogram, a pulse rate, a blood pressure, a body temperature, a transcutaneous arterial oxygen saturation (SpO2), a respiration rate, and an end-tidal carbon dioxide concentration (EtCO2). The monitor device 11 is configured to issue an alert. The monitor device 11 is an example of a medical device.

The assigning device 12 is a device that provides an input user interface for a medical worker 40 to input managing information described later. the assigning device 12 may be configured to display the physiological information such as a waveform and a numerical value that are acquired by the monitor device 11. In this example, the assigning device 12 is a mobile device that can be carried by the medical worker 40. Examples of the mobile device include a cellular phone, a portable information terminal, and the like. Examples of the portable information terminal include not only a general-purpose terminal device such as a smartphone and a tablet terminal, but also an exclusive input device.

As an alert is issued from the monitor device 11, the medical worker confirms the substance of the alert, and performs an appropriate management. The management includes a primary management such as stopping the issuance of an alert, and a secondary management such as confirming the condition of the subject 30 and the state of the medical equipment including the monitor device 11.

The managing information is indicative of whether the alert issued from the monitor device 11 is an actionable alarm or a non-actionable alarm. The "actionable alarm" means an alarm that notifies a condition that a therapeutic management or a non-therapeutic management is required. The "non-actionable alarm" means an alarm that notifies a condition that neither therapeutic nor non-therapeutic management is required.

Examples of the therapeutic managements include head-up, repositioning, cooling, aspiration of airway secretions, use of an inhaler or nebulizer, manual ventilation, alteration of oxygen flow, administration of as-needed medication or dietary supplement, and the like. Examples of the non-therapeutic managements include room visit, medical examination, vital sign measurement, blood glucose measurement, reattachment of sensor electrodes, feeding, soothing the patient to sleep, and the like.

The alert includes a vital alarm and a technical alarm. The vital alarm is an alarm that is generated in accordance with a change in the vital sign of the subject 30. The technical alarm is an alarm that is generated in accordance with a change in the medical device or the measurement condition for the subject 30.

Each of the vital and technical alarms includes a true alarm and a false alarm. The true alarm corresponds to a case where an event to be notified by the alarm coincides with an event that actually occurs. The false alarm corresponds to a case where the event to be notified by the alarm is different from the event that actually occurs. It should be noted that whether the state notified by the alarm is actionable or non-actionable may not be coincide with the fact that the alarm is true or false.

An example of a true actionable vital alarm corresponds to a case where an alarm indicating tachycardia is issued under a condition that tachycardia occurs in a patient with sepsis. Another example of the true actionable vital alarm corresponds to a case where an alarm indicating low SpO2 is issued under a condition that respiratory distress occurs in a certain patient.

An example of a true non-actionable vital alarm corresponds to a case where an alarm indicating bradycardia is issued in accordance with a respiratory variability that occurs in a certain patient. Another example of the true non-actionable vital alarm corresponds to a case where an alarm indicating tachycardia is issued under a condition that a pediatric patient is crying.

An example of a true actionable technical alarm corresponds to a case where an alarm indicating that a sensor electrode is detached is issued for a bedridden patient. An example of a true non-actionable technical alarm corresponds to a case where an alarm indicating sensor contact failure is issued due to a body movement of the patient.

An example of a false actionable vital alarm corresponds to a case where an alarm indicating ventricular tachycardia is issued for a patient undergoing convulsions.. An example of a false non-actionable vital alarm corresponds to a case where an alarm indicating cardiac arrest is issued under a condition that an electrocardiogram electrode is detached from a patient.

An example of a false actionable technical alarm corresponds to a case where an alarm indicating sensor detachment is issued under a condition that a sensor signal is too weak to perform measurement. An example of a false non-actionable technical alarm corresponds to a case where an alarm requesting a check is issued due to a temporary attachment failure of the sensor.

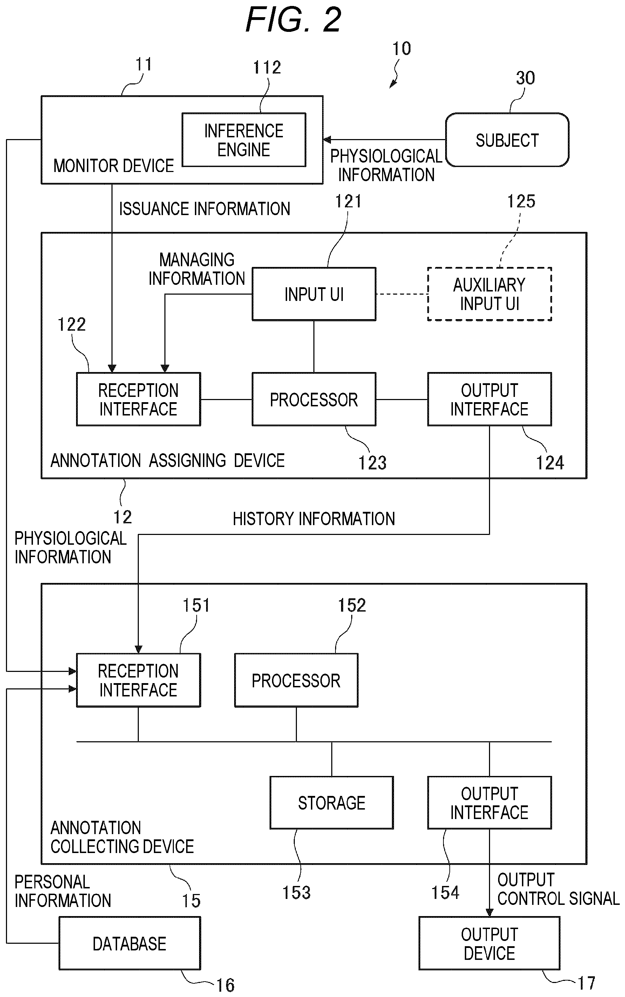

FIG. 2 illustrates a functional configuration of the assigning device 12. the assigning device 12 includes an input user interface 121, a reception interface 122, a processor 123, and an output interface 124.

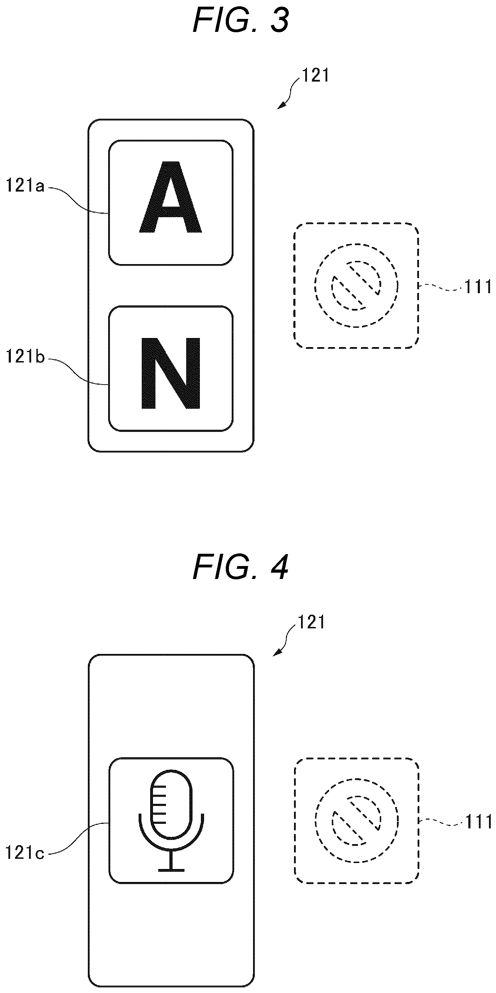

FIG. 3 illustrates an example of the input user interface 121. The input user interface 121 includes an actionable button 121a and a non-actionable button 121b. Each of the actionable button 121a and the non-actionable button 121b may be a button switch that can be mechanically actuated, or may be a button image that is displayed on a touch panel.

The actionable button 121a is a button for inputting, as the managing information, a notification that the alert issued from the monitor device 11 is an actionable alarm. The non-actionable button 121b is a button for inputting, as the managing information, a notification that the alert issued from the monitor device 11 is a non-actionable alarm. After the medical worker 40 stops an alert issued from the monitor device 11, the medical worker 40 inputs the managing information with the actionable button 121a or the non-actionable button 121b.

As illustrated in FIG. 2, the monitor device 11 is configured to output issuance information indicating that an alert is issued. The issuance information includes information such as a type of abnormality detected in the physiological information, a date and time when the alarm is issued, and the like. The issuance information may be in the form of digital data or analog data.

The reception interface 122 is configured as a hardware interface that receives the issuance information. In the case where the issuance information is in the form of analog data, the reception interface 122 includes an appropriate conversion circuit including an A/D converter.

The processor 123 is configured to enable the input user interface 121 to receive the managing information in response to the reception of the issuance information by the reception interface 122.

The input of the managing information to the input user interface 121 is not necessarily required to be performed by operating the button switch or the button image. Additionally or alternatively, the managing information may be inputted through a voice input or a gesture input. In this case, a word or gesture indicating that the alarm is an actionable alarm, as well as a word or gesture indicating that the alarm is a non-actionable alarm are determined in advance.

FIG. 4 illustrates another example of the input user interface 121 that allows voice input. As the processor 123 permits the acceptance of the managing information, for example, an icon 121c requesting the voice input is displayed or illuminated.

As illustrated in FIG. 2, the input user interface 121 is configured to output the managing information that is inputted by the medical worker 40. The managing information may be in the form of digital data or analog data.

The reception interface 122 is configured to receive the managing information. In the case where the managing information is in the form of analog data, the reception interface 122 includes an appropriate conversion circuit including an A/D converter.

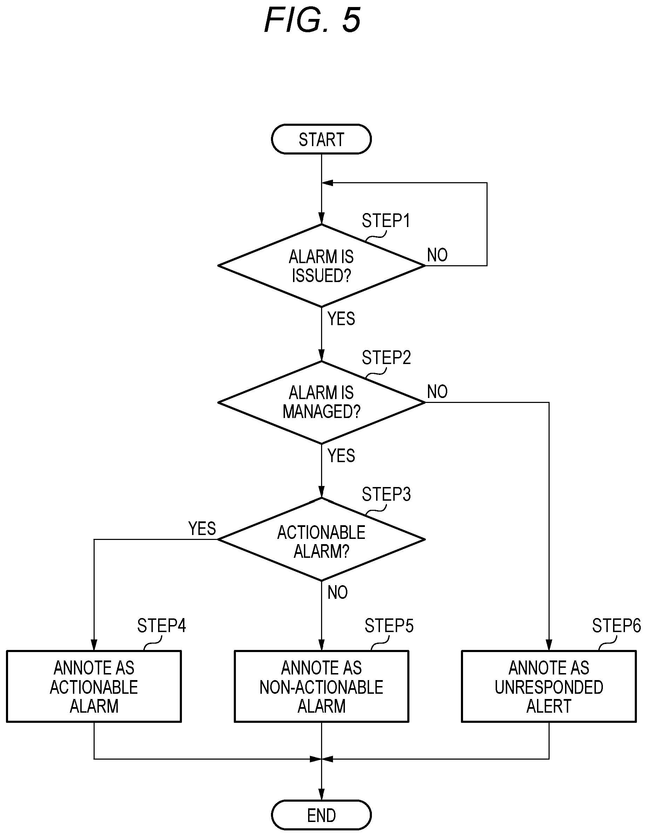

The processor 123 is configured to perform the annotation assignment processing illustrated in FIG. 5 in accordance with the managing information received by the reception interface 122. The annotation assignment processing is performed to associate an alert issued from the monitor device 11 with a management of the alert performed by the medical worker 40.

First, the processor 123 determines whether the reception interface 122 has received the issuance information from the monitor device 11 (STEP 1). The processing is repeated until it is determined that the issuance information is received (NO in STEP 1).

In response to the determination that the issuance information is received (YES in STEP 1), the processor 123 determines whether the reception interface 122 receives the managing information before a prescribed time period elapses after the issuance information is received (STEP 2).

In a case where it is determined that the managing information is received before the elapse of the prescribed time period (YES in STEP 2), the processor 123 determines whether the managing information indicates an actionable alarm (STEP 3).

In a case where it is determined that the managing information indicates an actionable alarm (YES in STEP 3), the processor 123 performs processing of assigning an annotation indicating an actionable alarm to the issuance information (STEP 4).

In a case where it is determined that the managing information does not indicate an actionable alarm (NO in STEP 3), the processor 123 performs processing of assigning an annotation indicating a non-actionable alarm to the issuance information (STEP 5).

As illustrated in FIG. 2, the processor 123 is configured to output, from the output interface 124, history information in which the annotation has been assigned to the issuance information. The output interface 124 is configured as a hardware interface.

The history information may be in the form of digital data or analog data. In the case where the history information is in the form of analog data, the output interface 124 includes an appropriate conversion circuit including a D/A converter.

As described above, even in a situation where an alarm indicating tachycardia is issued from the monitor device with respect to a certain subject, there would be a case where tachycardia that requires treatment actually occurs (actionable) and a case where no treatment is required because a temporary pulse increase is caused by crying or erroneous detection by the sensor (non-actionable). As long as the possibility of an actionable alarm is undeniable, medical workers are required to check how the alert is triggered. However, the frequency of the non-actionable alarm is higher than the frequency of the actionable alarm. As the number of sensors and medical devices for monitoring a subject increases, the number of managements to non-actionable alarms with a relatively low urgency tends to increase as well. Accordingly, the burden on medical workers known as "alarm fatigue" tends to increase.

Thus, it is required for the monitor device to have a capability of appropriately discriminating the actionable alarm from the non-actionable alarm, and suppressing the output of the latter. It is conceivable that the monitor device is provided with an inference engine having acquired a discrimination capability through machine learning. However, in order to prepare an inference engine having a highly accurate discrimination capability, an enormous amount of training data is required. The training data is, for example, a combination of physiological information that triggers an alert to be issued, and an annotation indicating whether the alert is an actionable alarm or a non-actionable alarm.

According to the configuration of the present embodiment, since the medical worker himself/herself who has managed the alert can assign an annotation to the alert through the input user interface, it is possible to prompt efficient collection of annotations having strongly reflected the monitoring environment of the physiological information in a workplace of the medical worker. Especially, since it is prompted to accumulate cases related to the non-actionable alarms issued frequently, it is possible to contribute to preparation of the high-quality training data. As a result, it is possible to reduce the burden imposed on the medical workers managing the alerts.

As illustrated in FIG. 5, in a case where the managing information is not received before the prescribed time period elapses after the issuance information is received (NO in STEP 2), the processor 123 assigns, to the issuance information, an annotation indicating that no response is performed to the alert (STEP 6). Namely, the alert corresponding to the issuance information is recorded as a non-responded alert.

Examples of situations where no response is performed to an alert include a case where the sensitivity of the medical worker to the alert is decreased due to a reason such as too many alerts, or a case where the medical worker is executing a task with a higher priority. The data assigned with the annotation indicating that no response is performed to the alert is also collected, whereby it is improved the contribution to the preparation of the training data for suppressing the issuance of the alarm with a relatively low urgency level.

As illustrated in FIG. 1, in addition to or in place of the mobile device carried by a medical worker 40, the monitor device 11 may serve as the assigning device 12. Namely, the input user interface 121 may be a part of the monitor device 11.

According to the above configuration, the medical worker who has performed the operation of inactivating the alert with respect to the monitor device 11 can directly attend to the assignment of the annotation. As a result, it is possible to reduce a mental burden that would be imposed on the medical worker who performs an additional work.

In this case, as illustrated in FIGS. 3 and 4, the input user interface 121 is preferably so arranged as to be adjacent to an inactivation user interface 111 provided in the monitor device 11 for inactivating the alert. According to the above configuration, it is possible to prompt a smooth transfer from the alert inactivating operation to the annotation assigning operation.

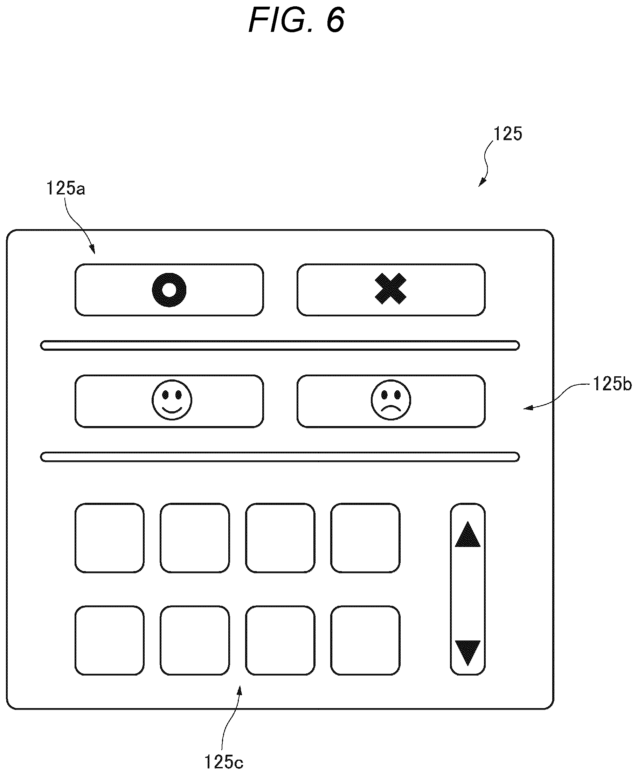

As illustrated in FIG. 6, the assigning device 12 may provide an auxiliary input user interface 125. The auxiliary input user interface 125 includes a first group 125a, a second group 125b, and a third group 125c. The medical worker 40 can input supplementary information for the alert with the auxiliary input user interface 125 after the assignment of an annotation to the alert with the input user interface 121.

The first group 125a includes a button or a button image for selectively inputting whether an alarm is true or false. As described above, the true alarm and the false alarm include an actionable alarm and a non-actionable alarm, respectively. Accordingly, by appending information indicating whether the alert assigned with the annotation is a true alarm or a false alarm, more detailed information about why and how the alert is triggered can be subjected to the machine learning. As a result, it is possible to realize inference of the alert type based on more detailed determinations.

The second group 125b includes buttons or button images for selectively inputting impressions of the medical worker 40 with respect to the alert. In this example, a button indicating an impression that the alert was useful, and a button indicating an impression that the alert was unnecessary are illustrated. By appending information indicating the impression of the medical worker 40 as to whether the alert associated with the annotation is appropriate, more detailed information about the contribution of the alert can be subjected to the machine learning. As a result, it is possible to realize inference of the alert type based on more detailed determinations.

The third group 125c includes a button or a button image for selectively inputting a type of the management performed by the medical worker 40 with respect to the alert. The type of the management includes a management that is appropriately selected from the therapeutic management and the non-therapeutic management described above. By appending information indicating what type of management is required for the actionable alarm or the non-actionable alarm corresponding to the annotation, more detailed information on the relationship between the alert and the management can be subjected to the machine learning. As a result, it is possible to realize inference of the alert type based on more detailed determinations.

In addition, since the auxiliary input user interface 125 is configured to selectively input information to be supplemented, it is possible to reduce a mental burden the medical worker 40 who performs additional work.

The auxiliary input user interface 125 may include at least one of the first group 125a, the second group 125b, and the third group 125c.

As illustrated in FIG. 1, the collecting system 10 may include an imaging device 131. The imaging device 131 is disposed so as to acquire an image in which at least one of the monitor device 11 and the subject 30 is captured. In other words, the imaging device 131 is so disposed as to be able to record what type of management is performed by the medical worker 40 with respect to the alert issued from the monitor device 11. The imaging device 131 may be installed in a specific medical device including the monitor device 11.

The collecting system 10 includes a display device 14 configured to display an image that is acquired by the imaging device 131 at least when an alert is issued. As long as image data can be received from the imaging device 131 over the communication network 20, the display device 14 may be an independent device, or may be a part of the monitor device 11 or the assigning device 12.

According to the above configuration, even in a place different from a medical site where the monitor device 11 is disposed, it is possible to refer to the image displayed on the display device 14 at an arbitrary timing after an alert is issued in order to assign an annotation to the alert. In other words, it is possible to alleviate the spatial and temporal constraints related to the annotation assigning operation. In addition, the annotation may be assigned by a person who is different from the medical worker operating in the medical site. Examples of such a person include an engineer who work in a medical-related company, a qualified medical professional on parental leave and the like.

In addition to or in place of the imaging device 131, the collecting system 10 may include a sound collecting device 132 for obtaining similar advantages. The sound collecting device 132 is disposed so as to acquire sound that is emitted from at least one of the monitor device 11 and the subject 30. In other words, the sound collecting device 132 is disposed so as to record what type of management is performed by the medical worker 40 with respect to the alert that is issued from the monitor device 11.

According to the above configuration, it is also possible to refer to the sound acquired by the sound collecting device 132 at any timing after an alert is issued, in a location different from the medical site where the monitor device 11 is installed, so that an annotation can be assigned to the alert. In other words, it is possible to alleviate the spatial and temporal constraints related to the annotation assigning operation.

As illustrated in FIG. 1, the collecting system 10 includes an annotation collecting device 15 (hereinafter, abbreviated as "a collecting device 15"). The collecting device 15 can communicate with the monitor device 11 and the assigning device 12 over the communication network 20. The collecting device 15 may be general-purpose or dedicated device disposed in a medical facility MF or outside the medical facility MF. The above-described display device 14 may be a part of the collecting device 15. The collecting device 15 may be a part of the monitor device 11 or a part the assigning device 12.

As illustrated in FIG. 2, the collecting device 15 includes a reception interface 151, a processor 152, a storage 153, and an output interface 154.

The reception interface 151 is configured as a hardware interface that receives the history information from at least one assigning device 12. In a case where the history information is in the form of analog data, the reception interface 151 includes an appropriate conversion circuit including an A/D converter. This description is similarly applied to other information or data that can be received by the reception interface 151 described later.

The processor 152 is configured to store the history information in the storage 153. The storage 153 may be implemented by a semiconductor memory, a hard disk drive, a magnetic tape drive, or the like.

The reception interface 151 is configured to be able to receive also the physiological information that is acquired by the monitor device 11. The reception interface 151 may receive the physiological information directly from the monitor device 11, or may receive physiological information that is copied by a mirroring hub device disposed on the communication network 20 from the physiological information transmitted from the monitor device 11 to another device. As used herein, the expression "physiological information acquired by the monitor device" means to include the latter case.

Examples of the physiological information include information indicating a value of a specific physiological parameter, information indicating a temporal change (waveform or trend) of the value, and the like. The physiological information is configured to include information indicating a time point at which the physiological information is acquired by the monitor device 11.

The processor 152 may be configured to acquire the physiological information at least when the monitor device 11 issues an alert, and to store the same in the storage 153 while associating with the history information that is created based on the alert.

For example, all the physiological information that are acquired by the monitor device 11 may be temporarily stored in a prescribed storage. The storage may be the storage 153 or a storage provided in a device on the communication network 20 capable of communicating with the collecting device 15. In this case, the processor 152 may specify a time point when the alarm is issued with reference to the history information, and acquire, from the storage, physiological information for a prescribed time period including the time point.

According to the above configuration, it is possible to automate the operation of associating an alert assigned with the annotation with the physiological information that triggers the alert. As a result, it is possible to improve the efficiency of the operation related to analysis of the history information described later, as well as preparation of data used for the machine learning of the inference engine 112 that is to be installed in the monitor device 11.

In addition to the above-described physiological information, a condition (e.g., sensor type) under which the physiological information is acquired by the monitor device 11 at least when an alert is issued, an alert issuance condition, or the like may be received by the reception interface 151.

As illustrated in FIG. 2, the collecting system 10 may include a database 16. The database 16 is for storing and managing electronic medical records. Although not illustrated, the database 16 can communicate with the collecting device 15 over the communication network 20. The database 16 may be disposed outside the medical facility MF.

As illustrated in FIG. 2, when an alert is issued from the monitor device 11, the processor 152 of the collecting device 15 may be configured to acquire personal information of the subject 30 from the database 16 through the reception interface 151. The personal information may include at least one of sex, height, weight, medical history, current symptom, and the like of the subject 30.

In this case, the processor 152 may be configured to associate the personal information with the history information that is created based on the annotation assigned to the alert by the assigning device 12, and to store them in the storage 153.

A background of issuance of the alert may be affected by personal properties of the subject 30. According to the above configuration, it is possible to create the history information based on consideration of such influence Accordingly, it is possible to improve the quality of data used for analysis of the history information described later, as well as the machine learning of the inference engine 112 that is to be installed in the monitor device 11.

In a case where a time point when a specific severe event (sudden death, ischemic stroke, myocardial infarction, vascular rupture, or the like) occurs can be specified in the electronic medical record stored in the database 16, and physiological information at the time when the severe event occurs can be acquired, an annotation indicating an actionable alarm may be assigned to the physiological information at any timing. In other words, an actual alert management need not be performed when an annotation indicating an actionable alarm is assigned. The personal information of the subject in whom the severe event occurred and the history information created based on the annotation are associated with each other and stored in the storage 153.

According to the above configuration, it is possible to assign an appropriate annotation with respect to a severe event with a low occurrence frequency, and it is possible to improve the issuance accuracy (specificity) of the actionable alarm.

The processor 152 of the collecting device 15 may receive the personal information of the medical worker 40 who inputted the managing information, through the reception interface 151. The personal information may be information that specifies, for example, an attribute of the medical worker 40. Examples of attributes include a doctor, a resident, a nurse, a job history, and the like. The personal information may be inputted through, for example, the input user interface 121 of the assigning device 12 or the auxiliary input user interface 125.

In this case, the processor 152 may be configured to associate the personal information of the medical worker 40 with the history information that is created in accordance with the annotation assigned to the alert by the assigning device 12, and to store them in the storage 153.

The quality of the annotation assigned with the assigning device 12 may be affected by the attribute of the medical worker 40 who inputted the managing information. According to the above configuration, it is possible to reflect such influence in the history information. Accordingly, it is possible to improve the quality of data used for analysis of the history information described later, as well as the machine learning of the inference engine 112 that is to be installed in the monitor device 11.

As illustrated in FIG. 2, the collecting system 10 may include an output device 17. The output device 17 is configured to visualize information as demanded. Examples of the output device 17 include a general-purpose device having a display, a printer, and a data output device. The output device 17 may be a part of the assigning device 12 or a part of the collecting device 15. As illustrated in FIG. 1, the output device 17 can communicate with the collecting device 15 over the communication network 20. Although not illustrated, the output device 17 may be disposed outside the medical facility MF.

The processor 152 of the collecting device 15 may be configured to create statistic information related to the annotation in accordance with the history information. For example, the statistic information may include a ratio of each number of annotations indicating actionable alarms, annotations indicating non-actionable alarms, and annotations indicating no response to the alert, to the total number of annotations.

The processor 152 may be configured to output, from the output interface 154, an output control signal that causes the output device 17 to output the statistic information. The output interface 154 is configured as a hardware interface. The output control signal may be a digital signal or an analog signal. In the case where the output control signal is an analog signal, the output interface 154 includes an appropriate conversion circuit including a D/A converter.

The above-described statistic information may be used in analysis of the operation state of a physiological information monitoring system in a medical site where the monitor device 11 is disposed. For example, in a case where the proportion of annotations indicating actionable alarms is high, it is implied an operation state where an abnormality of a subject with a relatively high necessity of management is appropriately notified by an alert.

On the other hand, in a case where the proportion of annotations indicating non-actionable alarms is high, it is implied a possible situation that the algorithm of the inference engine 112 installed in the monitor device 11 may not be matched with at least the actual situation of the medical site. In a case where the proportion of annotations indicating no response to the alerts is high, it is implied a possible situation that there may be a problem in the operation of the physiological information monitoring system.

Therefore, a user (medical worker, consultant, or the like) who has reviewed the statistic information can classify the unnecessary alerts into one caused by a deficiency of the algorithm and one caused by a deficiency of the operation environment. Accordingly, it is possible to discriminate a countermeasure for improving the algorithm from a countermeasure for improving the operation environment.

Improved algorithms and operating environments help to improve the quality of care that is to be performed to subjects and reduce the burden that would be imposed on the medical workers. Examples of measures for improving the algorithm include changing the alert issuance condition of the monitor device 11, retraining of the inference engine 112, and the like. Examples of measures for improving the operation environment include changing the alert issuance condition of the monitor device 11, a maintenance check of a sensor or a probe attached to the subject 30, changing the personnel allocation of the medical worker, and the like.

The processor 152 of the collecting device 15 may be configured to determine, in accordance with the history information stored in the storage 153, whether the alert issuance condition needs to be changed. For example, in a case where the number or the ratio of the unresponded alerts exceeds a prescribed threshold, it is determined that the alert issuance condition needs to be changed. Additionally or alternatively, in a case where the number or ratio of alarms that are determined to be unnecessary exceeds a prescribed threshold, it is determined that the alert issuance condition needs to be changed. Specifically, it is determined that a condition change (adjustment of a threshold value or the like) that suppresses the alert issuance is necessary.

In response to the determination that the alert issuance condition needs to be changed, the processor 152 outputs, from the output interface 154, an output control signal that causes the output device 17 to notify the determination. The output device 17 notifies the user that the alert issuance condition needs to be changed, through at least one of visual notification, audible notification, and haptic notification. The user who receives the notification changes the setting of the monitor device 11 as required.

By setting the alert issuance condition appropriately, both the algorithm and the operation environment can be improved as described above. Accordingly, it is possible to improve the quality of care that is to be performed to subjects and reduce the burden that would be imposed on the medical workers.

As described above, the monitor device 11 includes the inference engine 112. The inference engine 112 is an algorithm that is generated through machine learning using a neural network. The inference engine 112 is configured to output, in response to an input of the physiological information acquired from the subject 30, a probability that a specific abnormality occurs in the physiological information. In a case where the probability exceeds a threshold, the monitor device 11 issues an alert corresponding to the abnormality.

The inference engine 112 may be installed in a device that can perform data communication with the monitor device 11. The data communication may be performed over the communication network 20 illustrated in FIG. 1, or may be performed with short-range wireless communication.

The processor 152 of the collecting device 15 may be configured to determine whether the inference engine 112 needs to be retrained in accordance with the history information stored in the storage 153. For example, in a case where the number or the ratio of the unresponded alerts exceeds a prescribed threshold, it is determined that the inference engine 112 needs to be retrained. Additionally or alternatively, in a case where the number or ratio of alarms that are determined to be unnecessary exceeds a prescribed threshold, it is determined that the inference engine 112 needs to be retrained.

In response to the determination that the inference engine 112 needs to be retrained, the processor 152 outputs, from the output interface 154, an output control signal that causes the output device 17 to notify the determination. The output device 17 notifies the user that the inference engine 112 needs to be retrained, through at least one of a visual notification, an audible notification, and a haptic notification. The notified user retrains the inference engine 112 as required. At the time of the retraining, the history information that is stored in the storage 133 in association with the physiological information is used as the training data.

The algorithm of the inference engine 112 generated with the machine learning that is directed to overall optimization would not be always adapted to the operating environment of a particular medical site. On the other hand, it is highly probable that the history information stored in the storage 153 of the collecting device 15 reflects the actual situation of the medical site where the monitor device 11 installed with the inference engine 112 is disposed. Accordingly, by retraining the inference engine 112 with the history information as the training data, it is possible to obtain an alert output characteristic of the monitor device 11 that is highly adapted to the operation environment of the medical site.

The inference engine 112 need not be generated with the machine learning using a neural network. The inference engine 112 may be generated with other machine learning algorithms. Examples of other machine learning algorithms include the decision tree, the random forest, the support vector machine, and the like.

A coverage of the medical site from which the collecting device 15 collects the history information may be appropriately determined. The coverage may be determined as a unit of a hospital room, a unit of a ward, or a unit of a clinical department in a specific medical facility. The history information may be collected from multiple medical facilities capable of performing communication over the communication network 20. In the case where the history information is collected from the multiple medical facilities, it is preferable to determine the coverage such that the clinical departments coincide with each other.

As the number of sources of the history information increases, insufficient number of cases as to the alert management in each of the medical facilities can be supplemented from each other. In addition, it is possible to reduce the burden that would be imposed on each of the medical facilities to accumulation of the cases as to the alert management.

Similarly, the inference engine 112 of the monitor device 11 that has been retrained in accordance with the determination performed by the collecting device 15 need not to be restored only to the medical site where the monitor device 11 is disposed. The algorithm of the inference engine 112 that has been retrained in accordance with the operation environment in a certain medical site may be applied to other medical sites having similar operation environments. Only the data for the retraining may be shared.

According to the above configuration, it is possible to efficiently improve the adaptability of the algorithm of the inference engine 112 to the operation environment in a specific medical site. In addition, it is possible to reduce the burden that would be imposed on the medical site to accumulate the cases as to the alert management for improving the adaptability.

In a case where the history information is provided from multiple entities, the processor 152 of the collecting device 15 may be configured to acquire a contribution degree of a source entity of the history information. The contribution may be defined in accordance with various criteria. For example, the contribution degree may be specified in accordance with the number of times that the history information is provided, the contribution degree to the determination as to whether the alarm issuance condition needs to be changed, the contribution degree to the determination as to whether the inference engine 112 needs to be retrained, or the like. The processor 152 may output, from the output interface 154, an output control signal that causes the output device 17 to output the contribution as specified.

According to the above configuration, it is possible to construct a system in which a reward corresponding to the degree of contribution is provided to a source entity of the history information. Under such a system, an incentive acts on inputting the managing information. Accordingly, a virtuous cycle of collecting more history information can be expected.

Each of the processor 123 of the assigning device 12 and the processor 152 of the collecting device 15 having various functions described above may be implemented by at least one general-purpose microprocessor that operates in cooperation with at least one general-purpose memory. Examples of the general-purpose microprocessor include a CPU, an MPU, and a GPU. Examples of the general-purpose memory include a ROM and a RAM. In this case, a computer program for executing the above-described processing may be stored in the ROM. The ROM is an example of a non-transitory computer-readable medium having stored a computer program. The general-purpose microprocessor designates at least a part of the program stored in the ROM, loads the designated program in the RAM, and executes the above-described processing in cooperation with the RAM. The computer program may be pre-installed in the general-purpose memory, or may be downloaded from an external server over the communication network 20, and then installed in the general-purpose memory. In this case, the external server is an example of a non-transitory computer-readable medium having stored therein a computer program.

Each of the processor 123 of the assigning device 12 and the processor 152 of the collecting device 15 having various functions described above may be implemented by an exclusive integrated circuit capable of executing the above-described computer program, such as a microcontroller, an ASIC, and an FPGA. In this case, the above-described computer program is pre-installed in a memory element included in the exclusive integrated circuit. The memory element is an example of the non-transitory computer-readable medium having stored the computer program.

Each of the processor 123 of the assigning device 12 and the processor 152 of the collecting device 15 having various functions described above may also be implemented by a combination of a general-purpose microprocessor and an exclusive integrated circuit.

The various configurations described above are merely illustrative for facilitating understanding of the invention. Each of the illustrative configurations may be appropriately modified or combined with another illustrative configuration within the gist of the invention.

The medical device that issues the alert is not limited to the monitor device 11 that is disposed in the vicinity of the subject 30. As long as the physiological information of the subject 30 can be acquired and an alert can be issued in accordance with the abnormality, a central monitor disposed in a nurse center, or an appropriate inspection device may also be an example of the medical device.

The timing when the medical worker assigns an annotation to the alert with the input user interface may be arbitrary. For example, the annotation may be assigned when the medical worker manages an alert, or when no alert is issued. This is because any visit to the room (when no alert is issued) would be a better timing for the medical worker to assign the annotation than a busy time for the alert management.

When the medical worker assigns the annotation to the alert with the input user interface, the physiological information such as waveforms or metrics displayed in the assigning device 12 may be referred to.

## Claims

1. An annotation collecting system, comprising:
a medical device configured to issue an alert; and
an annotation assigning device configured to provide an input user interface arranged to input managing information indicating whether the alert is an actionable alarm or a non-actionable alarm, and to assign an annotation corresponding to the managing information to the alert.

2. The annotation collecting system according to claim 1,
wherein the annotation assigning device is configured to assign an annotation indicating that no response is performed with respect to the alert, in a case where the managing information is not inputted after the alert is issued and before a prescribed time period elapses.

3. The annotation collecting system according to claim 1 or 2,
wherein the input user interface is a part of the medical device.

4. The annotation collecting system according to claim 3,
wherein the input user interface is so arranged as to be adjacent to an inactivation user interface configured to inactivate the alert.

5. The annotation collecting system according to any one of claims 1 to 4,
wherein the annotation assigning device is configured to provide an input user interface arranged so as to allow a selective input of at least one of: whether the alert is true or false; a type of management performed by a medical worker with respect to the alert; and an impression of the medical worker as to whether the alert is appropriate.

6. The annotation collecting system according to any one of claims 1 to 5, further comprising:
an imaging device configured to acquire an image in which at least one of a subject and the medical device is captured; and
a display device configured to display the image at least when the alert is issued,
wherein the input user interface is configured to allow an input of the managing information while referring to the image.

7. The annotation collecting system according to any one of claims 1 to 6, further comprising:
a sound collecting device configured to collect sound emitted from at least one of a subject and the medical device,
wherein the input user interface is configured to allow an input of the managing information while referring to the sound.

8. The annotation collecting system according to any one of claims 1 to 7, further comprising:
an annotation collecting device configured to acquire history information assigned with the annotation from at least one annotation assigning device, and to store the history information as acquired.

9. The annotation collecting system according to claim 8,
wherein the annotation collecting device is configured to acquire physiological information of a subject at least when the alert is issued, and to associate the physiological information as acquired with the history information.

10. The annotation collecting system according to claim 8 or 9,
wherein the annotation collecting device is configured to acquire personal information of at least one of a subject and a person who inputted the managing information, and to associate the personal information as acquired with the history information.

11. The annotation collecting system according to any one of claims 8 to 10,
wherein the annotation collecting device is configured to cause an output device to output statistic information that is related to the annotation and is created in accordance with the history information.

12. The annotation collecting system according to any one of claims 8 to 11,
wherein the annotation collecting device is configured to determine whether an issuance condition of the alert needs to be changed in accordance with the history information.

13. The annotation collecting system according to any one of claims 8 to 12,
wherein the medical device is configured to determine whether abnormality is found in physiological information of a subject with an inference engine that has been subjected to machine learning in which a combination of the physiological information and the annotation is used as training data; and
wherein the annotation collecting device is configured to determine whether the inference engine needs to be retrained in accordance with the history information.

14. The annotation collecting system according to any one of claims 8 to 13,
wherein the annotation collecting device is configured to acquire a degree of contribution of a source entity that provides the history information.

15. An annotation collecting method, comprising:
providing an input user interface arranged to input managing information indicating whether an alert issued from a medical device is an actionable alarm or a non-actionable alarm; and
assigning an annotation corresponding to the managing information to the alert.
